Europäisches Patentamt

European Patent Office

Office européen des brevets

(11) Veröffentlichungsnummer: **0 059 404**
**A1**

(12) # EUROPÄISCHE PATENTANMELDUNG

(21) Anmeldenummer: 82101320.8

(22) Anmeldetag: 20.02.82

(51) Int. Cl.³: **C 07 C 19/02**
**C 07 C 17/16**

(30) Priorität: 26.02.81 DE 3107105

(43) Veröffentlichungstag der Anmeldung:
08.09.82 Patentblatt 82/36

(84) Benannte Vertragsstaaten:
**BE DE FR GB IT NL**

(71) Anmelder: HOECHST AKTIENGESELLSCHAFT
Postfach 80 03 20
D-6230 Frankfurt/Main 80(DE)

(72) Erfinder: Grünbein, Wolfgang, Dr.
Alt-Oberliederbach 35
D-6237 Liederbach(DE)

(72) Erfinder: Lendle, Wilhelm, Dr.
Hirschpfad 5
D-6232 Bad Soden am Taunus(DE)

(72) Erfinder: Post, Hendrik Willem
Paulinenweg 4
D-6238 Hofheim am Taunus(DE)

(72) Erfinder: Richter, Heinz
Wilhelm-Bonn-Strasse 2
D-6242 Kronberg/Taunus(DE)

(72) Erfinder: Rossberg, Manfred, Dr.
Am Rotfeld 8
D-6273 Waldems(DE)

(54) **Verfahren zur Gewinnung von Methylchlorid enthaltenden Gasen.**

(57) Methylchlorid enthaltende Gase werden aus Chlorwasserstoff enthaltenden Gasen und Methanol durch Reaktion in wasserhaltiger Zinkchlorid-Lösung bei erhöhter Temperatur hergestellt. Als Chlorwasserstoff enthaltendes Gas wird ein Gemisch der Zusammensetzung

| | | | | |
|---|---|---|---|---|
| $CH_3Cl + CH_4$ | 15 | - | 80 | Vol.- % |
| $N_2$ | 3 | - | 50 | % |
| HCl | 10 | - | 25 | % |
| $CCl_3H + CCl_4$ | 0,1 | - | 5 | % |
| $CH_2Cl_2$ | 5 | - | 20 | % |
| $Cl_2$ | 0 | - | 0,1 | % |

eingesetzt.

EP 0 059 404 A1

HOECHST AKTIENGESELLSCHAFT    HOE 81/F 042    C053404    Dr.SP/ss

Verfahren zur Gewinnung von Methylchlorid enthaltenden
Gasen

Die vorliegende Erfindung betrifft ein Verfahren zur Herstellung von Methylchlorid enthaltenden Gasen aus Chlorwasserstoff enthaltenden Gasen und Methanol in der Gasphase an einem flüssigen Kontakt.

Bei der technischen Chlorierung von Methan fällt neben den Chlorierungsprodukten, wie Methylchlorid oder Methylenchlorid, noch Chlorwasserstoff an, der aufgearbeitet werden muß. Das gleiche gilt für die Chlorierung von Methylchlorid, Methylenchlorid oder Chloroform. Neben der Elektrolyse von wäßrigem Chlorwasserstoff unter Rückgewinnung von Chlor ist vor allem die Reaktion des Chlorwasserstoffs mit Methanol von Interesse, da hierbei ohne Einsatz von Chlor und Methan ein technisches Methanchlorierungsprodukt erhalten werden kann.

Die Umsetzung von Chlorwasserstoff und Methanol, die sogenannte "Methanolveresterung", kann unter anderem in einer ca. 70 %-igen wäßrigen Zinkchlorid-Lösung vorgenommen werden. Nach Ullmann, Enzyklopädie der technischen Chemie, 3. Aufl., 1954, Bd. 5, S. 401 werden bei 130 - 150°C durch die Zinkchloridschmelze kontinuierlich nahezu äquimolare Mengen von Methanol und Chlorwasserstoff geleitet. Das entweichende rohe Methylchlorid nimmt das Reaktionswasser mit. Im Ausgas ist, neben nicht umgesetzten Ausgangsprodukten, als unerwünschte Verunreinigung noch Dimethylether vorhanden.

Ungünstigerweise ist der bei der Methanchlorierung anfallende Chlorwasserstoff nicht rein, sondern enthält mindestens äquimolare Mengen an Methanchlorierungsprodukten. In manchen Fällen wird bei der Chlorierung ein Inertgas, wie z.B. Stickstoff, eingeleitet, was zu einer weiteren Verringerung des Chlorwasserstoff-Gehalts der Reaktionsgase führt.

Die Abtrennung des Chlorwasserstoffs aus diesem Gemisch geschieht in der Technik meist durch Waschen mit Wasser und Austreiben von HCl aus der wäßrigen Absorptionslösung durch Erhitzen. Ferner kann man aus den Reaktionsprodukten der Methan- oder Methylchlorid-Chlorierung durch Auswaschen mit Chlormethanen und nachfolgende Verflüssigung Chlorwasserstoff gewinnen oder durch direkte Verflüssigung und fraktionierte Destillation unter Druck einen relativ reinen trockenen Chlorwasserstoff erhalten. Dieser Chlorwasserstoff kann zur Methanolveresterung eingesetzt werden. Beispielsweise kann gemäß DE-OS 18 06 988 ein Gasstrom, der aus 87 Mol-% HCl und 13 Mol-% Methylchlorid besteht, mit Methanol vermischt und in eine Methanolveresterungs-Vorrichtung eingeführt werden.

Es ist ein Nachteil dieses Verfahrens, daß der Methanolveresterung Trennoperationen vorgeschaltet werden müssen. Dies gilt umso mehr, wenn die Chlorierung von Methan (oder Methylchlorid) in Gegenwart von Inertgasen stattfindet.

Es bestand daher die Aufgabe, ein Verfahren zu finden, bei dem der Chlorwasserstoff der Methan- oder Methylchlorid-Chlorierung in Gegenwart von Stickstoff ohne vorherige Abtrennung für die Methanolveresterung eingesetzt werden kann.

Die vorliegende Erfindung löst diese Aufgabe.

Es wurde nun ein Verfahren zur Herstellung von Methylchlorid enthaltenden Gasen aus Chlorwasserstoff enthaltenden Gasen und Methanol durch Reaktion in wasserhaltiger Zinkchlorid-Lösung bei erhöhter Temperatur gefunden, das dadurch gekennzeichnet ist, daß man als Chlorwasserstoff enthaltendes Gas ein Gemisch folgender Zusammensetzung:

$$CH_3Cl + CH_4 \quad 15 \quad - \quad 80 \text{ Vol.-\%}$$
$$N_2 \qquad\qquad 3 \quad - \quad 50 \quad \%$$
$$HCl \qquad\qquad 10 \quad - \quad 25 \quad \%$$
$$CCl_3H + CCl_4 \quad 0,1 \quad - \quad 5 \quad \%$$
$$CH_2Cl_2 \qquad\qquad 5 \quad - \quad 20 \quad \%$$
$$Cl_2 \qquad\qquad\quad 0 \quad - \quad 0,1 \quad \%$$

einsetzt. Die Reaktionstemperatur liegt bei 60 - 200°C, insbesondere 70 - 180°C. Der Chlorgehalt des Reaktionsgases beträgt vorzugsweise 0,01 - 0,05 Vol.-%, der Gehalt an Methylchlorid vorzugsweise 25 - 80 Vol.-%.

Bei dem Verfahren des Standes der Technik liegt die Schmelze von wasserhaltigem Zinkchlorid in einem säurebeständigen Gefäß (Keramik oder säurefest ausgemauertes Stahlgefäß) vor. Nach einer besonderen Ausführungsform des erfindungsgemäßen Verfahren findet die Reaktion jedoch in einer Kolonne statt, die mit Zinkchlorid-Lösung berieselt wird.

Es ist besonders vorteilhaft in den unteren Teil der Kolonne Methanol und das Chlorwasserstoff enthaltende Gas einzuspeisen und am Kopf der Kolonne die Zinkchlorid-Lösung aufzugeben und das entstehende Gasgemisch abzuziehen. Dieses Gasgemisch enthält neben etwas Chlorwasserstoff und Dimethylether das gesamte Methylchlorid und das Reaktionswasser.

Die am Boden der Kolonne ablaufende Zinkchlorid-Lösung wird daher bevorzugt direkt wieder am Kopf der Kolonne aufgegeben, d.h. im Kreislauf geführt werden. Als Kolonne kann eine Füllkörper- oder Glockenbodenkolonne z.B. verwendet werden.

Um die Bildung von Dimethylether zu unterdrücken, ist es vorteilhaft ein Molverhältnis Methanol/HCl von unter 1:1, vorzugsweise im Bereich von 0,3:1 bis 0,95:1 zu wählen. Es ist aus dem gleichen Grunde günstig, Methanol auf einen Boden der Kolonne einzuspeisen, der unterhalb des Bodens

- 4 - 0059404

der Zugabe des HCl enthaltenden Gasgemisches angeordnet ist.

Es ist überraschend, daß durch diese Verfahrensweise ein erheblicher Teil des Chlorwasserstoffes des eingesetzten Gasgemisches mit Methanol zu Methylchlorid reagiert, ohne daß eine Abtrennung von HCl nötig würde und daß gleichzeitig der Dimethylether-Gehalt des resultierenden Gases um ein Vielfaches unter dem bei der technischen Methanolveresterung liegt.

Es ist zwar bekannt (Chemie-Ing.-Technik $\underline{42}$ (1970), S. 1215), daß bei der Umsetzung von $CH_3OH$ und HCl das Reaktionsgleichgewicht auf Seiten der Endprodukte liegt und die Reaktionsgeschwindigkeit nicht durch die Endprodukte beeinflußt wird. Diese Literaturstelle betrifft jedoch die Bildung von Methylchlorid an einem Feststoff-Katalysator aus $Al_2O_3$.

Die Erfindung wird durch die folgenden Beispiele näher erläutert.

Beispiele

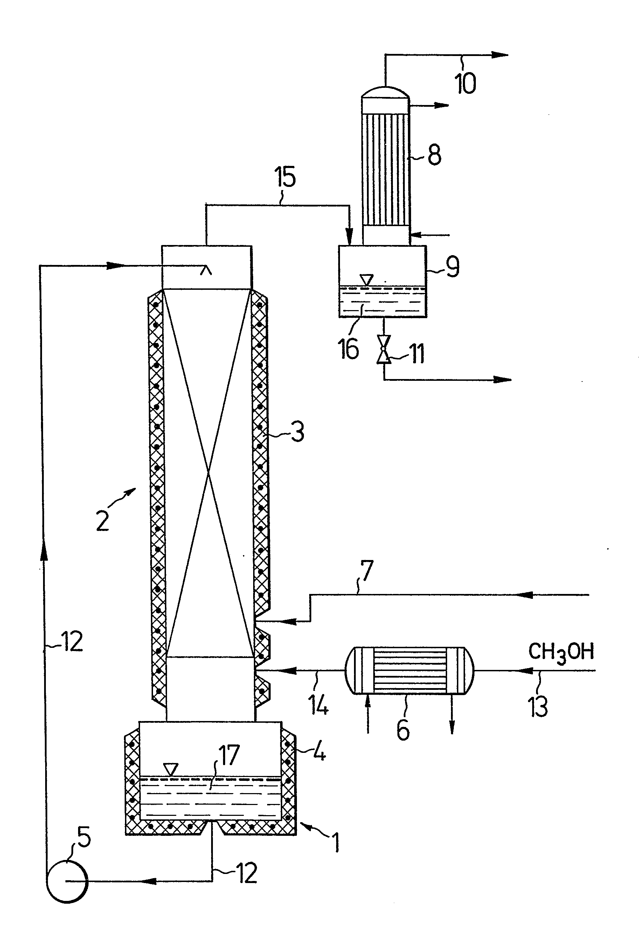

Die Versuchsapparatur wird in der Figur dargestellt. Die Reaktion findet in einer gläsernen Glockenbodenkolonne (Innendurchmesser 5 cm, eine Glocke pro Boden, 20 Böden) statt. Aus dem Sumpfgefäß (1) wird eine heiße, wäßrige Zinkchlorid-Lösung (17) über Leitung (12), in die Pumpe (5) eingefügt ist, entnommen und am Kolonnenkopf wieder aufgegeben. Über Leitung (13) wird flüssiges Methanol einem Verdampfer (6) zugeführt und dort verdampft. Das dampfförmige Methanol wird über Leitung (14) in der Nähe des untersten Bodens in die Glockenbodenkolonne (2) eingeleitet (selbstverständlich kann auf den Methanol-Verdampfer (6) verzichtet werden und Methanol in flüssiger Form eindosiert werden). Auf einem darüber angeordneten Boden der Kolonne wird über Leitung

(7) das aufzuarbeitende, Chlorwasserstoff enthaltende Gas aufgegeben. Es ist bevorzugt, wenn mindestens 3 Böden zwischen der Methanol-Zuführung und der Chlorwasserstoff-Zuführung vorhanden sind.

Kolonne (2) und Sumpfbehälter (1) tragen außen eine Isolierschicht (3 bzw. 4) wobei jedoch die Möglichkeit zu Kühlen oder zu Beheizen nicht ausgeschlossen wird. Während der Versuche wird die Kolonne durch schwache Beheizung von (3) bei 98°C Innentemperatur gehalten. Am Kopf der Kolonne (2) strömt das Gas, das an Chlorwasserstoff verarmt ist, über Leitung (15) in einen Kondensator (8), in den das Reaktionswasser kondensiert. Das Kondensat (16) enthält ferner etwas gelösten Chlorwasserstoff und restliches Methanol. Es sammelt sich im Behälter (9) und kann mittels Hahn (11) entnommen werden. Über Leitung (10) verlassen die nicht kondensierten Anteile des Reaktionsgases den Kondensator (8). Die weitere Aufarbeitung kann in einer Kondensation der Chlorkohlenwasserstoffe und nachfolgender (fraktionierter) Destillation bestehen.

Beispiel 1:

Pro Stunde werden 330 l eines Gases mit

| | | |
|------|-------|-----------|
| 19.1 | Vol.-% | $CH_3Cl$ |
| 8.3 | % | $CH_2Cl_2$ |
| 2.1 | % | $CHCl_3$ |
| 0.2 | % | $CCl_4$ |
| 14 | % | $HCl$ |
| 30 | % | $N_2$ |
| Rest | | $CH_4$ |

auf den vierten Boden (von unten gerechnet) der Glockenbodenkolonne gegeben. Eine wäßrige Lösung, die aus 30 Gew.-Teilen Wasser und 70 Gew.-Teilen Zinkchlorid hergestellt wurde, wird mit der Pumpe dem Sumpfgefäß entnommen, in einem Wärmetauscher (nicht gezeichnet) auf 80 - 85°C erhitzt und auf den Kopf der Kolonne aufgegeben (Pumpenleistung: 6 - 8 l/h). Auf den zweiten Boden der Kolonne wird flüssiges Methanol mit 56 ml/h eindosiert.

Bei der Kühlung des entstehenden Reaktionsgases auf 16°C fallen 33 ml eines Kondensates an, das 26,7 Gew.-% HCl und 0,9 % Methanol enthält. Der Rest besteht aus Wasser. Die verbleibende Gasphase hat folgende Zusammensetzung

| 28.9 | Vol.-% | $CH_3Cl$ |
|------|--------|----------|
| 8.4 | % | $CH_2Cl_2$ |
| 2.2 | % | $CHCl_3$ |
| 0.2 | % | $CCl_4$ |
| 1.4 | % | HCl |
| 30.4 | % | $N_2$ |
| 215 | ppm | Dimethylether |
| Rest | | $CH_4$. |

Beispiel 2:

Pro Stunde werden 260 l einer Gasmischung mit

| 39.9 | Vol.-% | $CH_3Cl$ |
|------|--------|----------|
| 12.9 | % | $CH_2Cl_2$ |
| 2.5 | % | $CHCl_3$ |
| 0.2 | % | $CCl_4$ |
| 15.2 | % | HCl |
| Rest | % | $N_2$ |

in den Gasraum des Sumpfgefäßes der Glockenbodenkolonne eingeleitet. Methanol wird mit 30 g/h dampfförmig zugegeben. Die restlichen Bedingungen entsprechen Beispiel 1. Man erhält 27,6 g Kondensat mit 29,6 Gew.-% HCl und 0,7 % Methanol, sowie ein Restgas mit folgender Zusammensetzung

50.1　　Vol.-%　$CH_3Cl$
13.5　　　%　　$CH_2Cl_2$
2.7　　　%　　$CHCl_3$
0.2　　%　　$CCl_4$
3.2　　　%　　$HCl$
15 ppm Dimethylether.

## Beispiel 3:

Pro Stunde werden 260 l einer Gasmischung mit

45.8　　Vol.-%　$CH_3Cl$
15.3　　　%　　$CH_2Cl_2$
2.9　　　%　　$CHCl_3$
0.2　　%　　$CCl_4$
16.5　　　%　　$HCl$
Rest　　　　　$N_2$

auf den 4. Boden der Kolonne gegeben. Methanol (30 g/h) wird dampfförmig zugeführt. Man erhält 27.6 g Kondensat mit 30.6 Gew.-% HCl und 0,28 % $CH_3OH$ (Rest: Wasser) sowie ein Restgas der Zusammensetzung

55.8　　Vol.-%　$CH_3Cl$
15.7　　　%　　$CH_2Cl_2$
3.1　　　%　　$CHCl_3$
0.2　　%　　$CCl_4$
4.3　　　%　　$HCl$
2 ppm Dimethylether
Rest　　　　　$N_2$.

## Patentansprüche:

1. Verfahren zur Herstellung von Methylchlorid enthaltenden Gasen aus Chlorwasserstoff enthaltenden Gasen und Methanol durch Reaktion in wasserhaltiger Zinkchlorid-Lösung bei erhöhter Temperatur, dadurch gekennzeichnet, daß man als Chlorwasserstoff enthaltendes Gas ein Gemisch der Zusammensetzung

| | | | |
|---|---|---|---|
| $CH_3Cl + CH_4$ | 15 | - 80 | Vol.-% |
| $N_2$ | 3 | - 50 | % |
| $HCl$ | 10 | - 25 | % |
| $CCl_3H + CCl_4$ | 0,1 | - 5 | % |
| $CH_2Cl_2$ | 5 | - 20 | % |
| $Cl_2$ | 0 | - 0,1 | %. |

einsetzt.

2. Verfahren nach Anspruch 1, dadurch gekennzeichnet, daß man die Reaktion in einer Kolonne durchführt, die mit wasserhaltiger Zinkchlorid-Lösung berieselt wird.

3. Verfahren nach Anspruch 2, dadurch gekennzeichnet, daß man in den unteren Teil der Kolonne das Chlorwasserstoff enthaltende Gas sowie Methanol einspeist und man am Kopf der Kolonne die Zinkchlorid-Lösung aufgibt und das entstehende Gasgemisch abzieht.

4. Verfahren nach Anspruch 2, dadurch gekennzeichnet, daß die Zinkchlorid-Lösung im Kreislauf geführt wird.

5. Verfahren nach Anspruch 1, dadurch gekennzeichnet, daß das Molverhältnis Methanol zu Chlorwasserstoff unter 1:1 liegt.

0059404

1/1

# 0059404

**Europäisches Patentamt**

## EUROPÄISCHER RECHERCHENBERICHT

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X,Y | <u>US - A - 3 502 733</u> (B.E.KURTZ u.a.) | | C 07 C 19/02 C 07 C 17/16 |
| | * Spalten 3-5; Figur * | 1,2,4 | |
| | -- | | |
| Y | <u>US - A - 2 847 484</u> (L.A.KOLKER) | | |
| | * Spalten 4-6; Figur * | 1,4 | |
| | -- | | |
| Y | <u>DE - C - 565 122</u> (I.G.FARBENINDUSTRIE) | | RECHERCHIERTE SACHGEBIETE (Int. Cl.³) |
| | * das ganze Dokument * | 1 | C 07 C 19/00 C 07 C 17/00 |
| | -- | | |
| A,D | <u>DE - A - 1 806 988</u> (STAUFFER CHEMICAL CO.) | | |
| | --------- | | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| DEN HAAG | 25. Mai 1982 | VAN GEYT |

EPA form 1503.1  06.78